(19) Europäisches Patentamt

**European Patent Office**

**Office européen des brevets**

(11) Numéro de publication: **0 265 470 B1**

(12) **FASCICULE DE BREVET EUROPEEN**

(45) Date de publication de fascicule du brevet: **02.09.92**  (51) Int. Cl.⁵: **A61N 5/06, A61B 17/36**

(21) Numéro de dépôt: **87902557.5**

(22) Date de dépôt: **24.04.87**

(86) Numéro de dépôt internationale :
**PCT/FR87/00139**

(87) Numéro de publication internationale :
**WO 87/06478 (05.11.87 87/24)**

(54) **INSTRUMENT DE TRAITEMENT SYSTEMATISE, UTILISANT NOTAMMENT L'ENERGIE LASER, UTILE PAR EXEMPLE EN DERMATOLOGIE.**

(30) Priorité: **30.04.86 FR 8606581**

(43) Date de publication de la demande:
**04.05.88 Bulletin 88/18**

(45) Mention de la délivrance du brevet:
**02.09.92 Bulletin 92/36**

(84) Etats contractants désignés:
**AT BE CH DE GB IT LI LU NL SE**

(56) Documents cités:
**EP-A- 0 172 490**
**FR-A- 2 357 008**

**Applied Optics, vol. 21, no. 19, 1 October 1982 (Optical Society of America, New York, USA), H. Fujii et al.: "Multispot laser photocoagulation system using a fiber bundle scanner", pp 3437-3442**

(73) Titulaire: **INSTITUT NATIONAL DE LA SANTE ET DE LA RECHERCHE MEDICALE (INSERM) 101, rue de Tolbiac F-75654 Paris Cédex 13(FR)**

(72) Inventeur: **BUYS, Bruno 6, rue Broca F-59000 Lille(FR)**
Inventeur: **SOZANSKI, Jean-Pierre 9A, rue P. Brossolette F-59239 Thumeries(FR)**
Inventeur: **MORDON, Serge Les Vignauds No 6 F-03260 Saint Germain des Fossés(FR)**
Inventeur: **BRUNETAUD, Jean-Marc 30, avenue Germaine F-59110 La Madeleine(FR)**
Inventeur: **MOSCHETTO, Yves Rue du Ouenes Ennetière-en-Weppe F-59320 Haubourdin(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

EP 0 265 470 B1

Optics and Laser Technology, vol. 14, no. 1, February 1982 (Butterworth & Co. Ltd., Guildford, GB), H. Fujii et al.: "Fibre bundle scanner for laser photocoagulation treatment", pp 39, 40

(74) Mandataire: **Lepage, Jean-Pierre**
**Cabinet Lepage & Aubertin Innovations et**
**Prestations S.A. 23/25, rue Nicolas Leblanc**
**B.P. 1069**
**F-59011 Lille Cédex 1 (Nord)(FR)**

## Description

L'invention est relative à un instrument de traitement systématisé. Elle trouvera notamment son application dans le domaine médical tel qu'en dermatologie pour le traitement des angiomes.

L'angiome plan, plus connu sous le nom "tache de vin", a pour origine une hypervascularisation du derme qui donne un effet inesthétique et disgracieux au niveau de la peau du sujet.

Actuellement, il est connu des méthodes de traitement de l'angiome plan qui consistent à obturer les vaisseaux sanguins au niveau de la zone concernée du derme de façon à obtenir une décoloration permanente de la lésion sans provoquer pour autant d'autres effets secondaires.

De nos jours, la technique du laser est généralement utilisé par le praticien qui réalise l'obturation des vaisseaux sanguins manuellement par photocoagulation à l'aide d'un laser du type Argon par exemple.

En effet, le laser Argon présente un rayonnement adapté aux traitements des angiomes plans car il est bien absorbé par les tissus vivants avec une action préférentielle au niveau des pigments rouges et noirs. C'est pourquoi on utilise souvent les effets thermiques du laser Argon pour le traitement de tels symptômes.

Dans le cas du traitement des angiomes, les effets thermiques souhaités sont une température comprise entre 60 et 80 °C qui doit être limitée aux micro-vaisseaux du derme afin d'éviter toute nécrose tissulaire entrainant des risques cicatriciels.

Actuellement, ce procédé de traitement est mis en oeuvre manuellement par le praticien qui, au moyen d'une source laser Argon munie d'un obturateur atténuateur commandé pour régler le temps d'irradiation et par l'intermédiaire d'une fibre optique transmettant l'énergie laser, effectue des tirs laser pour former une multitude d'impacts voisins au niveau de la zone à traiter.

Dans ce cas l'habileté du praticien est primordiale car c'est lui qui maitrise tous les paramètres du traitement à savoir la puissance du rayonnement, le temps d'exposition, le positionnement relatif des différents impacts et l'uniformité de ces impacts.

Le succès du traitement dépend donc essentiellement du praticien et de son habileté à maitriser ces paramètres qui peuvent déterminer des effets thermiques très variés tant par la température atteinte qui entraine soit une coagulation soit une volatilisation, que par la localisation de cette élèvation de température.

De plus, à ces paramètres, vient s'ajouter un élément supplémentaire directement lié à la puissance de l'impact réalisé, il s'agit d'un effet de diffusion thermique autour de l'impact réalisé qui provoque une accumulation thermique qu'il faudra également maitriser.

Par ailleurs, le praticien n'ayant d'autres moyens de repérage des impacts que le positionnement d'un nouvel impact par rapport à celui qui vient d'être réalisé ceci afin de juxtaposer les impacts de façon contigue, le phénomène de diffusion thermique oblige le praticien à effectuer l'impact suivant contigu qu'après un certain temps de relaxation pour éviter de prendre en compte les effets d'accumulation thermique et par conséquence éviter une surcharge en température.

Ces différents paramètres pris en considération font que l'efficacité actuelle du traitement représente environ 50 à 60 % de succès et obligent les patients à faire appel à des praticiens très expérimentés car le succès en est essentiellement dépendant.

De plus, les angiomes occupent la plupart du temps des surfaces importantes de l'ordre de plusieurs centimètres carrés qui ne sont pas toujours planes et étant donné que le diamètre des spots laser élémentaires au niveau des impacts est de l'ordre du millimètre, la mise en oeuvre du traitement obligera le praticien à juxtaposer manuellement plusieurs centaines de tirs sans pratiquement aucune référence de positionnement.

En effet, du fait de la technique mise en oeuvre, le praticien est soumis au port de lunettes de protection et ainsi il ne perçoit que faiblement le spot qu'il positionne. Ceci rend également difficile l'uniformité des diamètres des spots effectués qui dépend essentiellement de la distance de la fibre optique par rapport à la zone sous investigation.

Le cumul de ces impératifs fait de ce procédé de traitement un procédé délicat et fastidieux à mettre en oeuvre qui de plus doit être fractionné en plusieurs séances pour ménager d'une part le patient qui doit être immobile pendant le traitement et d'autre part le praticien qui doit soutenir une attention accrue.

Pour pallier aux échecs dans les résultats de cette thérapeutique et pour faciliter le travail du praticien, il a été envisagé d'automatiser sa tâche en robotisant l'intervention.

En effet, il a été présenté il y a quelques années une installation de traitement constituée essentiellement par un bras robotisé effectuant le déplacement contrôlé d'une pièce à main dans laquelle est disposée la fibre optique.

Cette méthode n'a jusqu'à présent fait l'objet d'aucun développement car sa mise en oeuvre est délicate car elle suppose une reconnaissance de formes traditionnelles et ne prend pas en compte notamment les mouvements du patient en cours de traitement.

Une telle installation facilite l'intervention du praticien mais donne des résultats qui sont inférieurs à ceux obtenus par la méthode traditionnelle manuelle.

D'autre part, il est également connu un instrument à main, réalisé sur le même principe que le dispositif précité, positionné par le praticien, c'est-à-dire à l'aide d'une fibre optique incluse dans un boîtier portatif transmettant l'énergie d'une source laser, mais dont le spot élémentaire a été élargi en utilisant une optique anamorphique.

Toutefois, avec cet instrument, l'uniformité du spot est difficile à obtenir, et de plus la profondeur de champ est très réduite. Par ailleurs, pour respecter les paramètres de traitement précités, il est fait appel à une source laser de forte puissance ce qui handicap son développement en augmentant le coût de revient d'un tel système.

On connaît également du document FR-A-2.357.008 un instrument médical de traitement thérapeutique, apte à être connecté à une source de rayonnement laser et comprenant des moyens de traitement d'une zone d'un corps.

L'instrument présente un bâti équipé d'un ensemble électro-mécanique, dans lequel le faisceau laser est canalisé, ainsi que des moyens de positionnement relatif du faisceau laser vis-à-vis de la zone à traiter.

L'ensemble électro-mécanique est constitué d'un assemblage de miroirs ou prismes permettant de concentrer le faisceau et de le diriger sur l'objet à irradier suivant trois coordonnées.

Il est à noter dans ce cas que le dispositif de balayage prévu est très encombrant et qu'il utilise une technique très lourde à mettre en oeuvre. Il faut également remarquer que l'énergie laser issue du dispositif de balayage est envoyée à distance vers le patient, et tout mouvement du patient va compliquer le traitement, comme évoqué ci-dessus.

On connaît du document "Applied Optics, Volume 21, No 19, 1er Octobre 1982; Fujii et al., "Multispot laser photocoagulation system using a fiber bundle scanner", pages 3437-3442" une méthode de traitement thérapeutique dans laquelle on met en oeuvre un faisceau de fibres pour véhiculer l'énergie laser.

Plus précisément, le faisceau de fibres se présente à son entrée sous la forme d'une nappe horizontale, devant laquelle le rayon laser est déplacé, puis à son autre extrémité se termine en toron pour former un "multispot".

La structure d'un tel appareil impose d'une part des surdimensionnements importants de la pièce à main et d'autre part n'est pas sans poser de nombreux problèmes au niveau du dispositif de positionnement du rayon laser devant chaque fibre de la nappe.

Le but de la présente invention est de proposer un instrument de traitement systématisé notamment utilisable en dermatologie pour le traitement des angiomes, qui permet de prendre le relais de l'habileté du praticien pour réduire le temps de traitement.

Un des buts d'un mode de réalisation particulier de la présente invention est de proposer un instrument de traitement systématisé, qui permette d'augmenter sensiblement le pourcentage de réussite du traitement des angiomes par rapport aux méthodes connues actuellement.

Un autre but d'un mode de réalisation particulier de la présente invention est de proposer un instrument de traitement systématisé qui permette le traitement des angiomes par zones géométriques parfaitement connues et reproductibles et qui, par un seul positionnement de l'instrument, traite idéalement et efficacement de zones de surface allant jusque 2,6 cm² alors que la fibre optique utilisée n'autorise généralement qu'un spot élémentaire d'environ 1 mm de diamètre.

Un autre but d'un mode de réalisation particulier de la présente invention est de proposer un instrument de traitement systématisé, notamment utilisable en dermatologie pour le traitement des angiomes plans, qui permette de maîtriser les paramètres thermiques en contrôlant la puissance d'irradiation, la distance instrument impact, le temps d'exposition et qui simultanément permette de maîtriser les paramètres géométriques à savoir l'uniformité des spots élémentaires et leur juxtaposition précise de façon contigue.

Un autre but d'un mode de réalisation particulier de la présente invention est de proposer un instrument de traitement systématisé qui permette de s'affranchir du temps de relaxation thermique crée par la diffusion de la chaleur au niveau de l'impact et par suite d'éviter toute surcharge en température.

Un autre but d'un mode de réalisation particulier de la présente invention est de proposer un instrument de traitement systématisé notamment utilisable en dermatologie pour le traitement d'angiomes, qui par un mode de balayage séquentiel programmé autorise de tirs laser consécutifs dont les impacts ne soient pas contigus tout en effectuant une juxtaposition finale des dits impacts très précise et contigue pour couvrir toute la zone du corps sous investigation.

Selon la présente invention, l'instrument de traitement systématisé, notamment utilisable en dermatologie pour le traitement des angiomes plans, apte à être connecté à une source de rayonnement laser, comprenant des moyens de traitement d'un corps et présentant : un bâti équipé d'un ensemble électro-mécanique, dans lequel le faisceau laser est canalisé, ainsi que des moyens de positionnement relatif du faisceau laser vis-à-vis de la zone sous-investigation, permettant d'une part le déplacement et l'orientation du faisceau laser vers la zone sous investigation, et d'autre part de former un spot élémentaire au niveau de l'impact avec la zone sous-investigation, est caractérisé par le fait qu'il comporte :

- une fibre optique, disposée dans le dit bâti, et assujettie au dit ensemble électro-mécanique, la fibre étant au moins immobilisée en un point fixe du bâti et son deuxième extrémité étant mobile,

- un dispositif mécanique apte à déplacer séquentiellement la dite deuxième extrémité de la fibre optique, en abscisse et en ordonnée repérées, afin d'engendrer un balayage séquentiel de la dite zone et le positionnement repéré du spot élémentaire,

- des moyens d'obturation commandés et synchronisés avec le dit dispositif mécanique pour contrôler le temps d'exposition de chaque tir, indépendamment des conditions de fonctionnement de la source laser,

afin d'autoriser une distribution des tirs laser telle qu'au moins deux spots élémentaires conséquents ne soient pas contigus afin d'éviter les effets d'accumulation thermique de l'impact précédent.

La présente invention sera mieux comprise à la lecture de la description suivante accompagnée des dessins en annexe qui en font partie intégrante.

La figure 1 représente une vue schématique en perspective d'un mode de réalisation de l'instrument de traitement systématisé de la présente invention qui permet d'en illustrer le principe de fonctionnement.

La figure 2 montre une disposition préférentielle des zones unitaires de traitement formées par la juxtaposition de spots élémentaires contigus selon la présente invention.

La figure 3 est une vue en coupe d'un mode de réalisation plus détaillé d'un instrument de traitement systématisé réalisé selon l'invention.

La figure 4 montre une vue schématique partielle de dessous de l'instrument représenté à la figure 3 qui montre la modularité de la zone unitaire traitée.

La figure 5 illustre un mode de balayage de la fibre optique de l'instrument de traitement de la présente invention autorisant une séquence de spots élémentaires consécutifs et contigus.

La figure 6 représente un autre mode de balayage préférentiel qui illustre une séquence de tirs laser consécutifs formant des spots élémentaires conséquents non contigus, toutefois la juxtaposition finale présentant alors des impacts contigus.

La figure 7 montre une vue de détail de l'instrument de traitement de la présente invention qui illustre ses moyens de dialogue et d'affichage.

La figure 8 montre une implantation possible de l'instrument de traitement systématisé de la présente invention sur une potence de façon à faciliter son utilisation par le praticien.

La présente invention vise un instrument de traitement systématisé dont l'application majeure sera médicale notamment en dermatologie pour le traitement des angiomes plans.

Toutefois, il est à remarquer qu'il pourrait être utilisé pour toute autre application de traitement d'une zone d'un corps nécessitant ces effets.

De plus, le terme "corps" est à prendre dans son sens large c'est-à-dire qu'il peut être formé par des tissus vivants ou par toute autre matière inerte. De plus, par "zone sous investigation" repérée 2 sur les figures, il faut entendre la surface unitaire qui est soumise aux effets de l'instrument de traitement systématisé de l'invention lors d'un positionnement.

L'instrument de traitement systématisé de la présente invention comprend des moyens de traitement d'une zone d un corps qui utiliseront les effets thermiques d'une source laser afin d'obtenir, dans le cas des angiomes plans, l'obturation des vaisseaux sanguins du derme par photocoagulation.

Pour mettre en oeuvre ce procédé de traitement d'une manière fiable et pour optimaliser le résultat obtenu, il est impératif de maitriser parfaitement certains paramètres fondamentaux qui sont d'une part des paramètres thermiques et d'autre part des paramètres géométriques.

En effet, rappelons que pour maitriser le procédé de traitement, il est nécessaire de contrôler la puissance d'irradiation, le temps d'exposition, et de tenir compte de l'effet de diffusion thermique pour éviter toute surcharge de température au niveau des impacts. Dans le cas des angiomes plans, ces différents paramètres thermiques doivent aboutir à une focalisation précise au niveau du vaisseau à coaguler et la température de chauffage doit être comprise entre 60 et 80 °C pour éviter tout risque cicatriciel préjudiciable.

En ce qui concerne les paramètres géométriques, il s'agit en fait de contrôler l'uniformité des spots émis, et leur juxtaposition précise afin que la zone unitaire sous investigation soit complètement couverte par une multitude d'impacts contigus de tirs laser consécutifs.

Par ailleurs, il est nécessaire de définir la dimension de cette zone sous investigation selon le cas à traiter pour d' une part tenir compte de la géométrie de la zone sous investigation et d'autre part éviter un inconfort au patient pendant le traitement.

Dans le cas de l'angiome plan, qui généralement occupe une surface importante de l'ordre de plusieurs centimètres carrés, la taille maximum de la zone élémentaire 2 à traiter sera donc volontairement limitée pour ces raisons.

En effet, un angiome peut avoir dans les trois dimensions une cartographie très tourmentée et variée, comme par exemple dans le cas d'une joue et aile du nez. On limitera donc la taille de la zone unitaire 2 afin que l'on puisse considérer la surface traitée plane pour préserver une distance de tirs constante. Ceci permettra également de réduire le temps d'immobilisation du patient à des valeurs raisonnables inférieures à la minute.

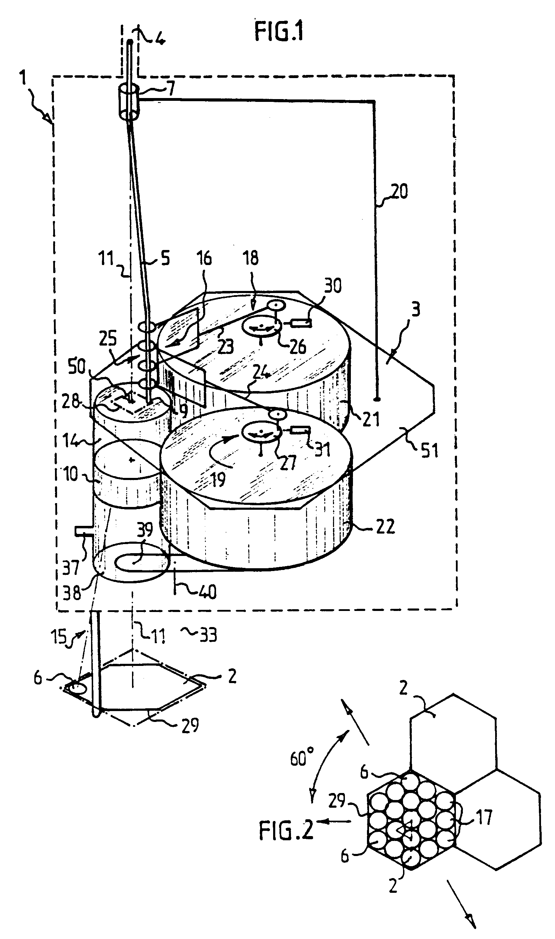

Afin de couvrir la totalité de la lésion, le praticien au lieu de juxtaposer les spots élémentaires manuellement selon la méthode traditionnelle, n'aura plus qu'à juxtaposer manuellement ces surfaces unitaires 2 comme le montre notamment la figure 2. Ceci permettra de réduire le nombre de positionnements de façon très importante et de diminuer considérablement le temps du traitement.

Selon la présente invention d'un mode de réalisation particulier, l'instrument de traitement systématisé 1 permet donc de répondre aux objectifs précités et d'automatiser le traitement de façon fiable et contrôlée à l'intérieur d'une surface unitaire 2.

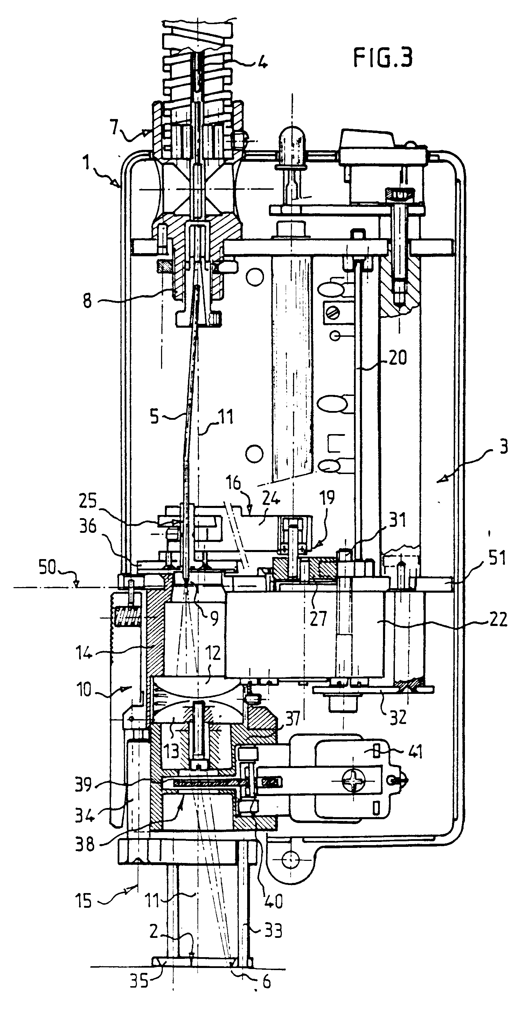

Afin de faciliter la compréhension, la réalisation de l'instrument de traitement systématisé 1 de la présente invention va être faite à la vue des figures 1 et 3 qui illustrent respectivement le principe et une réalisation préférentielle détaillée de la présente invention.

L'instrument de traitement systématisé 1 est constitué autour d'un bâti 3 formé d'une platine support d'appareillage 51 et d une potence 20 et servant de support aux différents constituants. Plus précisément, dans ce bâti est disposée en outre une fibre optique 5 permettant de transmettre l'énergie lasser et de former finalement un spot élémentaire 6 au niveau de l'impact avec la zone sous investigation 2. Pour ce l'instrument est notamment connecté à une source de rayonnements laser, non représentée, connexion repérée par 4 sur les figures.

Jusqu'à présent, une telle réalisation est connue de l'Homme de l'Art et on utilisera une fibre optique dont le diamètre permettra l'obtention finale d'un spot élémentaire 6 d'un diamètre compris entre 0,5 et 2 mm.

La fibre optique 5 sera avantageusement au moins immobilisée en un point fixe 7 du bâti 3 qui comprendra un système de centrage et d'immobilisation 8 du type presse-étoupe.

Par ailleurs, l'extrémité 9 de la fibre optique 5, montée sur le bâti 3, qui peut constituer une source de rayonnement laser mobile de faible inertie est placée en regard d'une optique de focalisation 10, l'ensemble fibre optique 5 et optique de focalisation 10 définissant alors un axe principal optique 11.

En ce qui concerne la réalisation proprement dite de l'optique de focalisation 10, elle formera un objectif constitué de deux lentilles plans convexes 12, 13 formant un doublet symétrique selon les techniques classiques centré sur l'axe principal 11. Le doublet est immobilisé axialement et solidaire du bâti 3 par la monture 14.

Il est à remarquer que de par cette disposition on reforme l'image inversée de l'extrémité 9 de la fibre optique. De plus, son ouverture sera telle que le faisceau issu de la fibre soit intercepté pour couvrir toute la surface unitaire 2 à traiter.

Par ailleurs, selon la présente invention, l'instrument de traitement 1 comporte essentiellement d'une part des moyens de positionnement 15 relatif de la fibre optique 9 vis-à-vis de la zone du corps sous investigation 2, et d'autre part des moyens de balayage séquentiel 16 de la dite zone sous investigation qui permettent de contrôler la position de l'extrémité de la figure optique 9 et du spot élémentaire 6 conséquent ainsi que la juxtaposition finale des différents impacts contigus 17 formés en optimalisant l'ordre de distribution des tirs laser pour qu'au moins lors de deux tirs laser consécutifs les spots élémentaires 6 conséquents ne soient pas contigus afin d'éviter les effets d'accumulation thermique de l'impact précédent.

Plus particulièrement, comme le montre la réalisation préférentielle des figures 1 et 3, les moyens de balayage séquentiel 16 sont constitués par un dispositif mécanique déplaçant séquentiellement l'extrémité 9 de la fibre optique 5 dans un plan 50 perpendiculaire à l'axe principal optique 11 afin d'engendrer le déplacement et le positionnement repéré du spot élémentaire 6 sur toute la surface unitaire sous investigation 2 ceci notamment par l'intermédiaire de l'optique de focalisation 10 précité. Ainsi, on pourra effectuer une suite de tirs laser séquentiellement afin de couvrir toute la surface unitaire sous investigation 2.

Le dispositif mécanique engendrant ce déplacement biaxial est formé notamment par deux systèmes bielle manivelle 18 et 19 tous deux solidaires du bâti 3 et montés sur la platine 51; on forme donc un système fixe par rapport au bâti 3, le point fixe 7 de la fibre optique et les bielles manivelles 18 et 19 étant réunis par l'intermédiaire d'une potence 20.

Les deux systèmes bielle-manivelle 18 et 19 sont chacun entraînés par un organe moteur respectivement 21 et 22 coopérant entre eux au niveau des extrémités de chaque bielle, respectivement repérées 23 et 24, pour constituer une articulation commune 25 à laquelle est solidarisée l'extrémité 9 de la fibre optique 5.

Afin de déplacer la fibre optique 5 en abscisse et en ordonnée repérées dans le plan 50 en formant une zone sensiblement semblable à la zone sous-investigation 2, les organes moteur 21, 22 de chaque système bielle-manivelle 18, 19 sont commandés indépendamment et leur rotation est contrôlée pour engendrer le balayage séquentiel par l'extrémité mobile 9 de la fibre optique de toute la surface correspondante à la surface unitaire de traitement 2.

Ainsi, si des angles de rotation sont imposés aux manivelles respectivement repérées 26 et 27, on engendre un mouvement et un positionnement relatif de l'articulation 25 par rapport à l'axe principal optique 11. De plus, si leur vitesse angulaire relative et absolue est imposée, la trajectoire du mouvement et sa vitesse sont également définies.

Ainsi la figure du chemin des déplacements a la forme d'un pseudo-losange 28 dont les côtés sont des portions de cercle. Il est à remarquer que dans cette forme, on pourra notamment circonscrire un hexagone 29 formé par l'ensemble des positions réellement exploitées.

La réalisation de ce système sera tel que les impératifs optiques soient respectés et notamment on s'assurera qu'une portion de l'extrémité 9 de la fibre optique 5 soit sensiblement parallèle à l'axe optique 11 pour cela on dédoublera avantageusement l'articulation 25 comme le montrent les figures 1 et 3. De plus, on s'assurera également que la flexion imposée à la fibre optique ne soit pas préjudiciable pour éviter une fatigue mécanique ainsi que les pertes optiques.

Ainsi, les moyens de balayage séquentiel 16 forment une structure isostatique dans laquelle la fibre optique joue le rôle d'un équipage mobile vis-à-vis du bâti 3.

En ce qui concerne l'entrainement proprement dit des systèmes bielle-manivelle 18, 19, les organes moteur 21 et 22 se présentent sous la forme de moteur "pas à pas" alimentés par des niveaux analogiques de courant prédéterminés permettant de diviser les "pas standards" des moteurs en plusieurs "micro-pas" afin d'augmenter la précision de positionnement.

A titre d'exemple non limitatif, on divise linéairement les pas principaux en 32 micro-pas entraînant une résolution de positionnement de 0,02 mm référé aux spots élémentaires de 1 mm ce qui autorisera une résolution largement suffisante dans l'application du traitement considéré.

Par ailleurs, la rotation angulaire de chaque moteur 21, 22 étant relative, des séquences d'initialisation absolues sont nécessaires pour contrôler leur positionnement. A cet égard, chaque système bielle-manivelle 18, 19 est asservi à un capteur de position, respectivement repéré 30, 31 autorisant une initialisation périodique du positionnement des organes moteur 21, 22.

En ce qui concerne la réalisation pratique de ce dispositif d'initialisation, les capteurs utilisés seront des capteurs de position fixes vis-à-vis du bâti 3 et positionnés en regard d'un index disposé sur les manivelles 26, 27. De plus, il est à remarquer que cet ensemble de capteurs ne nécessite aucune précision car il ne repère que des positions correspondantes à chaque séquence répétée soit par exemple tous les quatre pas car on retrouve dans ce cas un état électrique identique pour le moteur.

Il est à noter que de ce fait on évite l'utilisation de capteurs d'avertissement continu tels que des capteurs potientiométriques ou des codeurs optiques plus difficiles à mettre en oeuvre et plus coûteux.

Par contre, le calage angulaire mécanique des moteurs 21 22 doit être parfait. On effectuera ce dernier par bridage 32 des moteurs sur la platine 20 pour les immobiliser vis-à-vis du bâti 3, ceci selon des techniques connues.

En ce qui concerne les moyens de positionnement 15 relatif de la fibre optique 5 et de son optique de focalisation 10 vis-à-vis de la zone du corps sous investigation 2, ils seront constitués par un ensemble d'entretoises 32, fixé sous le bâti 3 et centré sur l'axe principal optique 11. Ainsi, on prendra appui sur la zone du corps 2 et on pourra fixer la distance de tirs et par conséquent la taille et l'uniformité prévues des spots élémentaires 2.

De plus, cet ensemble d'entretoises 33 présentera des moyens de réglage 34 pour adapter la distance de tirs à la valeur souhaitée. Une telle réalisation mécanique est à la portée de l'Homme de l'Art.

Par ailleurs, pour faciliter le positionnement par le praticien des différentes surfaces unitaires correspondantes aux zones sous investigation 2, comme le montre la figure 2, on pourra prévoir un support indépendant adhésif, non représenté, préalablement appliqué sur l'épiderme du patient, ce support comportant des repères de positionnement visibles adaptés à la forme géométrique de la surface unitaire 2. Alors, l'ensemble d'entretoises 33 présentera avantageusement une fenêtre découpée, au niveau du derme, qui facilitera ce positionnement vis-à-vis du repérage adhésif.

Par ailleurs, selon la présente invention d'un mode de réalisation particulier, on contrôle la puissance d'irradiation de la source laser et à cet égard l'instrument de traitement 1 présente des moyens de mesure de la puissance du rayonnement laser émis.

Ces moyens de mesure sont basés sur le principe de la sphère intégratrice qui est une méthode fidèle de mesure indirecte de puissance totale rayonnée.

En ce qui concerne leur réalisation pratique, ils sont constitués par une pseudo-sphère intégratrice, formée par la monture 14 support de l'optique de focalisation 10, obturée au niveau de la source par un couvercle mobile 36 et en amont du doublet 12, 13 par l'obturateur 38, dont la description sera donnée ultérieurement.

La monture 14 sera réalisée telle que l'ensemble des surfaces de la cavité soit réfléchissant et diffusant du rayonnement spécifique du laser. Une partie du rayonnement total diffusé est prélevée et mesurée par un capteur photoélectrique 37 qui sera avantageusement muni d'un filtre adapté au laser. De plus, la fibre optique 5 sera positionnée pendant la mesure coaxialement à l'axe principal optique 11 et la mesure proprement dite sera réalisée et traitée par des techniques à la portée de l'Homme de l'Art.

Enfin, en ce qui concerne la partie optique proprement dite de l'instrument à main de la présente invention, ce dernier comprend des moyens d'obturation 38 commandés et synchronisés avec les dits moyens de balayage 16 pour contrôler le temps d'exposition de chaque tir indépendamment des conditions de fonctionnement de la source laser.

Plus précisément, l'obturateur 38 est constitué d'une palette 39, mobile angulairement sur un axe 40 parallèle à l'axe principal optique 11. Elle est entrainée par un système mécanique à bras de levier par un électro-aimant 41 par exemple. Une telle réalisation est notamment connue de l'Homme de l'Art.

Toutefois, le plan d'obturation a été choisi dans une zone afocale privilégiée constituée par le pseudo-foyer des faisceaux générés par la nature diaphragmée de la source objet. De plus, la palette est constituée en matière réfléchissante diffusante telle qu'en alliage d'aluminium.

Par ailleurs, pour gérer le fonctionnement automatique des différents moyens qui viennent d'être décrits, l'instrument 1 de la présente invention selon un mode de réalisation préféré comporte une unité de commande automatique du dit instrument organisée autour d'une unité centrale électronique, d'une base de temps et de circuits annexes qui autorisent en outre le contrôle des moyens de balayage, la mesure de la puissance de l'irradiation, la commande des moyens d'obturation synchronisée, le dialogue avec l'opérateur.

Ces différents éléments électroniques seront constitués par des composants électroniques tels que micro-processeur, mémoire vive, mémoire morte et autres circuits intégrés dont la mise en oeuvre est à la portée de l'Homme de l'Art considéré.

Toutefois en ce qui concerne le circuit de contrôle des moyens de balayage, il comporte au moins une mémoire dans laquelle sont introduites des données afin d'autoriser une séquence de tir laser pour couvrir par leurs impacts 17 une figure géométrique 29 par juxtaposition de spots élémentaires 6 contigus parfaitement reproductible dans le temps.

A cet égard, pour faciliter ultérieurement la répartition manuelle par le praticien des surfaces unitaires 2, la figure géométrique se présente avantageusement sous la forme d'un hexagone régulier, leur jointure mutuelle étant parfaite par six axes différents à 60 degrés. De plus, l'hexagone est la continuité géométrique du triangle de base constitué par trois spots 6 circulaires contigus entre eux.

On a remarqué de bons résultats en formant un hexagone dont la diagonale est comprise entre 3 et 20 mm, formé par juxtaposition de spots élémentaires de diamètre compris entre 0,5 et 2 mm.

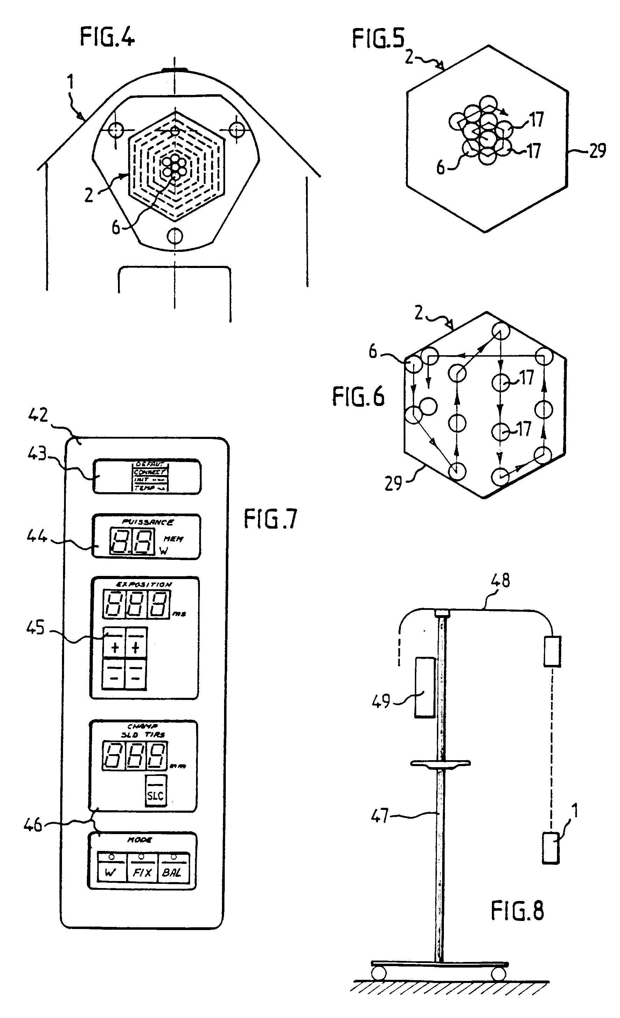

De plus, l'électronique de commande est réalisée telle que par l'intermédiaire du circuit de dialogue et du circuit de contrôle des moyens de balayage, la surface de la figure géométrique est modulable comme le montre particulièrement la figure 4. A cet égard, on autorisera par exemple une sélection de six champs se répartissant et autorisant notamment des hexagones de diagonales comprises entre 3 et 20 mm, chaque champ étant centré sur l'axe optique principal 11.

Un des aspects original de la présente invention selon un mode de réalisation préféré concerne l'ordre de distribution automatique des tirs laser afin de couvrir à la fin d'une séquence de tir complètement la surface unitaire de la zone sous investigation 2 par une juxtaposition contigue des différents spots élémentaires 6 émis lors de la séquence.

Toutefois, au cours de cinéthermographies d'un tir laser isolé élevant la température au niveau de l'impact de 60 à 80 ° C, on a relevé un temps de relaxation thermique de l'ordre de 1,5 secondes après ce tir avec une diffusion de température confinée sur un diamètre de 3 à 4 mm au niveau du derme.

Ainsi des tirs juxtaposés répétitifs dans cette zone de diffusion et avant relaxation induisent un phénomène d'accumulation et par conséquent une surcharge en température.

Ce phénomène est négligeable dans le cas d'une pratique manuelle telle que l'effectue actuellement le praticien car les temps de positionnement sont largement supérieurs aux temps de relaxation thermique. Par contre, lors d'un balayage automatique très rapide, des tirs juxtaposés répétitifs nécessitent une temporisation entre chaque tir pour qu'il y ait relaxation.

Toutefois, ce phénomène d'accumulation thermique augmente le temps nécessaire pour traiter une zone définie, c'est pourquoi la présente invention propose un mode de balayage optimisé afin de couvrir la surface unitaire sous investigation dans un temps de cycle inférieur à la minute et même voisin de 40 secondes.

Ce mode de balayage autorisé par les moyens de balayage séquentiel de la présente invention permet au moins de réaliser deux tirs laser consécutifs sans pour autant que les spots élémentaires 6 conséquents ne soient contigus ceci afin d'éviter les effets d'accumulation thermique dus à la diffusion de la température et à la périphérie d'un impact. Néanmoins à l'issue de cette séquence tous les impacts 17 seront juxtaposés et parfaitement contigus les uns les autres.

A ce sujet, la figure 6 montre un ordre de balayage séquentiel par lequel, les spots élémentaires 6 ayant un diamètre sensiblement voisin d'un millimètre, les spots successifs sont alors toujours éloignés d'au moins deux millimètres. Ceci permet de mettre en oeuvre des temps de répétition de tirs d'une valeur moyenne de 140 millisecondes et par suite de réduire considérablement le temps du traitement d'une surface élémentaire 2.

Dans ce cas, l'unité de commande automatique du système et son circuit de contrôle des moyens de balayage sont programmés par un logiciel qui permet de repérer en abscisse et en ordonnée les différentes positions des spots élémentaires 6 et de déplacer la fibre optique à cet égard par des rotations contrôlées des organes d'entrainement 21 et 22 du système de balayage 16. Ce mode de balayage illustré est tout à fait arbitraire et d'autres modes de balayage pourraient être envisagés par programmations différentes.

Toutefois, il est à remarquer que l'unité de commande comporte des moyens pour annihiler le balayage séquentiel de tirs laser formant des spots disjoints pour autoriser soit un balayage automatique de spots contigus consécutifs comme le montre la figure 5 soit un balayage manuel traditionnel.

En outre, pour introduire les différentes données et déclencher le mode d'utilisation souhaité, le praticien dispose d'un tableau d'affichage et de commande 42, tel que représenté à la figure 7, qui sera avantageusement séparé de l'instrument de traitement 1 proprement dit afin d'éviter un encombrement trop important. Il en est de même d'ailleurs pour la localisation de l'unité de commande précité.

Ce tableau de commande 42 permettra par exemple de disposer de voyants tests du fonctionnement interne du système en 43, de contrôler la mesure de puissance par un affichage en 44, de contrôler le temps d'exposition et d'introduire ces données en 45, de sélectionner le type de balayage et de choisir l'ampleur du champ de traitement en 46. Ces différents éléments sont réalisés selon des techniques courantes de l'électronique.

Enfin, pour faciliter l'intervention du praticien lors du traitement, l'instrument de traitement 1 de la présente invention sera avantageusement supporté par la flèche 48 d'une potence 47, l'unité centrale électronique et le tableau de commande 42 étant disposés sur le mât de la potence 47 notamment en 49 sur la figure 8.

## Revendications

1. Instrument de traitement systématisé (1), notamment utilisable en dermatologie pour le traitement des angiomes plans, apte à être connecté à une source de rayonnement laser 4, comprenant des moyens de traitement d'un corps (2) et présentant : un bâti (3) équipé d'un ensemble électro-mécanique (16), dans lequel le faisceau laser est canalisé, ainsi que des moyens de positionnement (15) relatif du faisceau laser vis-à-vis de la zone sous-investigation (2), permettant d'une part le déplacement et l'orientation du faisceau laser vers la zone sous-investigation (2), et d'autre part de former un spot élémentaire (6) au niveau de l'impact (17) avec la zone sous-investigation (2), caractérisé par le fait qu'il comporte :

   - une fibre optique (5), disposée dans le dit bâti (3), et assujettie au dit ensemble électro-mécanique (16), la fibre étant au moins immobilisée en un point fixe (7) du bâti (3) et son extrémité (9) étant mobile,
   - un dispositif mécanique (18-27) apte à déplacer séquentiellement la dite extrémité (9) de la fibre optique, en abscisse et en ordonnée repérées, afin d'engendrer un balayage séquentiel de la dite zone et le positionnement repéré du spot élémentaire (6),
   - des moyens d'obturation (38) commandés et synchronisés avec le dit dispositif mécanique (18-27) pour contrôler le temps d'exposition de chaque tir, indépendamment des conditions de fonctionnement de la source laser,
   afin d'autoriser une distribution des tirs laser telle qu'au moins deux spots élémentaires (6) conséquents ne soient pas contigus afin d'éviter les effets d'accumulation thermique de l'impact précédent.

2. Instrument de traitement selon la revendication 1, caractérisé par le fait qu'il comprend des moyens de mesure de la puissance du rayonnement laser émis pour contrôler la puissance d'irradiation.

3. Instrument de traitement selon la revendication 1, caractérisé par le fait qu'il comporte une optique de focalisation (10), en regard de l'extrémité (9) de la fibre optique, définissant avec celle-ci un axe principal optique (11), et que le dispositif mécanique (18-27) déplace l'extrémité (9) de la fibre optique (5) dans un plan (50) perpendiculaire au dit axe principal optique (11).

4.  Instrument de traitement selon la revendication 3, caractérisé par le fait que le dit dispositif mécanique est formé par deux systèmes biellemanivelle (18, 19), solidaires du bâti (3) et entraînés chacun par un organe moteur (21, 22), coopérant entre eux au niveau des extrémités de chaque bielle (23, 24), pour constituer une articulation commune (25) à laquelle est solidarisée l'extrémité (9) de la fibre optique (5) afin de la déplacer dans un plan (50) en abscisse et en ordonnée repérées.

5.  Instrument de traitement selon la revendication 4, caractérisé par le fait que les organes moteur (22, 22) de chaque système bielle-manivelle (18, 19) sont commandés indépendamment et leur rotation est contrôlée pour engendrer le balayage séquentiel par l'extrémité mobile (9) de la fibre optique (5) d'une zone semblable (50) à la zone sous-investigation (2).

6.  Instrument de traitement selon la revendication 4, caractérisé par le fait que les organes moteur (21, 22) des systèmes bielle-manivelle (18, 19) se présentent sous la forme de moteurs "pas à pas" alimentés par des niveaux analogiques de courant prédéterminés permettant de diviser les "pas standards" en plusieurs "micro-pas" pour augmenter la précision de positionnement.

7.  Instrument de traitement selon la revendication 4, caractérisé par le fait que chaque système bielle-manivelle (18, 19) est asservi à un capteur de position (30, 31) autorisant une initialisation périodique de positionnement des organes moteur (21, 22).

8.  Instrument de traitement selon la revendication 1, présentant une optique de focalisation (10) immobilisée par une monture (14), portée par le bâti (3), et placée dans l'axe principal optique (11) sous l'extrémité (9) de la fibre optique (5), caractérisé par le fait que les moyens de positionnement (15) relatif de la fibre (5) et de son optique de focalisation (10) vis-à-vis de la zone du corps sous investigation (2) sont constitués par un ensemble d'entretoises (33), fixé sur le bâti (3) et centré sur l'axe principal optique (11), prenant appui sur la zone du corps (2), présentant des moyens de réglage (34) pour adapter la distance de tir et par conséquent la taille et l'uniformité des spots élémentaires (6).

9.  Instrument de traitement selon la revendication 2, présentant une optique de focalisation (10) immobilisée par une monture (14), portée par le bâti (3), et placée dans l'axe principal optique (11) sous l'extrémité (9) de la fibre optique (5), caractérisé par le fait que les moyens de mesure du rayonnement laser sont constitués par une pseudo-sphère intégratrice formée par la monture (14) support de l'optique de focalisation (10), autorisant la mesure indirecte de la puissance totale rayonnée, réfléchissant et diffusant le rayonnement laser dont une partie est prélevée et mesurée par un capteur photoélectrique (37) adapté au laser.

10. Instrument de traitement selon la revendication 1, caractérisé par le fait qu'il comporte une unité de commande automatique (49) du dit instrument (1) organisée autour d'une unité centrale électronique, d'une base de temps et de circuits annexes qui autorisent en outre le contrôle du dit dispositif mécanique (18-27) de balayage, la mesure de la puissance de l'irradiation, la commande des moyens d'obturation synchronisés, le dialogue avec l'opérateur.

11. Instrument de traitement selon la revendication 10, caractérisé par le fait que le circuit de contrôle du balayage comporte au moins une mémoire dans laquelle sont introduites des données afin d'autoriser une séquence de tirs laser pour couvrir par leurs impacts (17) une figure géométrique (29), par juxtaposition de spots élémentaires contigus (6), parfaitement reproductible dans le temps.

12. Instrument de traitement selon la revendication 11, caractérisé par le fait que la surface de la figure géométrique (29) est modulable par l'intermédiaire du circuit de dialogue et du circuit de contrôle du balayage.

13. Instrument de traitement selon la revendication 11, caractérisé par le fait que la figure géométrique (29) se présente sous la forme d'un hexagone régulier dont la diagonale est comprise entre 3 et 20 mm formé par juxtaposition de spots élémentaires (6) de diamètre compris entre 0,5 et 2 mm.

14. Instrument de traitement selon la revendication 10, caractérisé par le fait que l'unité de commande (49) comporte des moyens pour annhiler le balayage séquentiel de tirs laser formant des spots (6) disjoints pour autoriser soit un balayage automatique de spots contigus consécutifs soit un balayage manuel.

**Claims**

1. Systematized treatment instrument (1), usable in particular in dermatology for the treatment of portwine naevuses, suitable for connection to a source of laser radiation (4), comprising means for treating a body (2) and having: a frame equipped with an electro-mechanical assembly (16), wherein the laser beam is channelled, as well as means (15) for relatively positioning the laser beam in respect of the area under investigation (2), permitting, on one hand, the displacement and orientation of the laser beam towards the area under investigation (2) and, on the other hand, the formation of an elementary spot (6) in the region of the impact (17) with the area under investigation (2), characterized by the fact that it comprises:
   - an optical fibre (5), disposed in the said frame (3) and secured to the said electro-mechanical assembly (16), the fibre being at least immobilized at a fixed point (7) on the frame (3) and its end (9) being mobile,
   - a mechanical device (18-27) suitable for sequentially displacing the said end (9) of the optical fibre, along a referenced abscissa and a referenced ordinate, in order to generate sequential scanning of the said area and permit the referenced positioning of the elementary spot (6),
   - shutter means (38) controlled and synchronized with the said mechanical device (18-27) for controlling the exposure time of each shot, whatever the laser source operating conditions may be, in order to allow the laser shots to be distributed such that at least two consequent elementary spots (6) are not contiguous, in order to avoid the effects of heat accumulation from the preceding impact,

2. Treatment instrument according to claim 1, characterized by the fact that it comprises means for measuring the power of the laser radiation emitted in order to control the irradiation power.

3. Treatment instrument according to claim 1, characterized by the fact that it comprises focussing optics (10) opposite the end (9) of the optical fibre, defining therewith an optical main axis (11), and that the mechanical device (18-27) displaces the end (9) of the optical fibre (5) in a plane (50) perpendicular to the said optical main axis (11).

4. Treatment instrument according to claim 3, characterized by the fact that the said mechanical device is formed by two connecting rod-crankshaft systems (18, 19), integral with the frame (3) and each driven by a motor means (21, 22), cooperating with one another in the area of the ends of each rod (23, 24), to form a common articulation (25) to which is secured the end (9) of the optical fiber (5) in order to displace it in a plane (50) along the referenced abscissa and ordinate.

5. Treatment instrument according to claim 4, characterized by the fact that the motor means (21, 22) of each rod-crankshaft system (18, 19) are operated independently and their rotation is controlled to generate sequential scanning by the mobile end (9) of the optical fibre (5) of an area (50) similar to the area under investigation (2).

6. Treatment instrument according to claim 4, characterized by the fact that the motor means (21, 22) of the rod-crankshaft systems (18, 19) take the form of "stepping" motors supplied by predetermined analogue current levels enabling the "standard steps" to be divided into a plurality of "micro-steps" to increase positioning accuracy.

7. Treatment instrument according to claim 4, characterized by the fact that each rod-crankshaft system (18, 19) is slaved to a position sensor (30, 31) permitting periodic initialization of positioning for the motor means (21, 22).

8. Treatment instrument according to claim 1, having focussing optics (10) immobilized by a mounting (14) carried by the frame (3) and placed on the optical main axis (11) below the end (9) of the optical fiber (5), characterized by the fact that the means (15) for relatively positioning the fibre (5) and its focussing optics (10) in respect of the area of the body under investigation (2) are formed by an assembly of distance pieces (33), fixed to the chassis (3) and centred on the optical main axis (11), bearing on the area of the body (2), having adjusting means (34) for adapting the firing distance and, consequently, the size and uniformity of the elementary spots (6).

**9.** Treatment instrument according to claim 2, having focussing optics (10) immobilized by a mounting (14), carried by the chassis (3), and placed on the optical main axis (11) below the end (9) of the optical fibre (5), characterized by the fact that the laser radiation measuring means are constituted by a pseudo-integrating sphere formed by the mounting (14) supporting the focussing optics (10), permitting indirect measurement of the total radiated power, reflecting and diffusing the laser radiation, a part of which is sampled and measured by a photoelectric sensor (32) adapted to the laser.

**10.** Treatment instrument according to claim 1, characterized by the fact that it comprises a unit (49) for automatically controlling the said instrument (1) organized around an electronic central unit, a time base and ancillary circuits which further permit control of the said mechanical scanning device (18-27), measurement of irradiation power, control of the synchronized shutter means and dialogue with the operator.

**11.** Treatment instrument according to claim 10, characterized by the fact that the scanning control circuit comprises at least a memory into which data is entered in order to permit a sequence of laser shots to cover with their impacts (17) a geometrical figure (29) through the juxtaposition of contiguous elementary spots (6), perfectly reproducible in time.

**12.** Treatment instrument according to claim 11, characterized by the fact the surface of the geometrical figure (29) can be modulated via the dialogue circuit and the scanning control circuit.

**13.** Treatment instrument according to claim 11, characterized by the fact that the geometrical figure (29) takes the form of a regular hexagon the diagonal of which measures between 3 and 20 mm, formed by the juxtaposition of elementary spots (6) having diameters of between 0,5 and 2 mm.

**14.** Treatment instrument according to claim 10, characterized by the fact that the control unit (49) comprises means for disabling the sequential scanning of laser shots forming disjoined spots (6) to permit either automatic scanning of consecutive contiguous spots or manual scanning.

**Patentansprüche**

**1.** Systematisiertes Behandlungsinstrument (1), das nämlich in der Dermatologie zur Behandlung der flachen Angiome einsetzbar und geeignet ist, an eine Laserstrahlungsquelle (4) angeschlossen zu werden, das Mittel zum Behandeln eines Körpers (2) und ein mit einer elektromechanischen Einheit (16) versehenes Gestell (3) umfaßt, in dem das Laserstrahlenbüschel geführt wird, sowie Mittel (15) zum Positionieren des Laserstrahlenbüschels bezüglich des Bereichs unter Untersuchung (2), die es erlauben, einerseits, das Laserstrahlenbüschel zu dem Bereich unter Untersuchung (2) hin zu verstellen und zu orientieren und, andererseits, einen Elementarabtastfleck (6) im Bereich des Treffpunktes (17) mit dem Bereich unter Untersuchung (2) zu bilden, dadurch gekennzeichnet, daß es

- eine im genannten Gestell (3) angeordnete und der genannten elektromechanischen Einheit (16) unterworfene optische Faser (5), wobei die Faser mindestens an einem festen Punkt (7) des Gestells (3) unbeweglich gemacht und deren Ende (9) beweglich ist,
- eine mechanische Vorrichtung (18-27), die geeignet ist, das genannte Ende (9) der optischen Faser sequenziell in festgelegter Abszisse und Ordinate zu verstellen, um eine sequenzielle Abtastung des genannten Bereichs und die festgelegte Positionierung des Elementarabtastflecks (6) zustandezubringen,
- mit der genannten mechanischen Vorrichtung (18-27) gesteuerte und synchronisierte Verschlußmittel (38) zum Einstellen der Ausstellungszeit jedes Schußes, unabhängig vom Betriebszustand der Laserstrahlenquelle,

umfaßt, um eine derartige Verteilung der Laserstrahlschüße zu erlauben, daß mindestens zwei nacheinanderfolgende Elementarabtastflecke (6) nicht neben einander liegen, damit die Wärmeakkumulierungswirkungen des vorigen Aufschlags vermieden wird.

**2.** Behandlungsinstrument nach Anspruch 1, dadurch gekennzeichnet, daß es Mittel zum Messen der ausgestrahlten Laserstrahlungskraft umfaßt, zum Überwachen der Bestrahlungskraft.

**3.** Behandlungsinstrument nach Anspruch 1, dadurch gekennzeichnet, daß es eine Bündelungsoptik (10) gegenüber dem Ende (9) der optischen Faser umfaßt, die mit diesem letzten eine optische Hauptachse (11) definiert, und daß die mechanische Vorrichtung (18-27) das Ende (9) der optischen Faser (5) in einer senkrecht zur genannten optischen Hauptachse (11) verlaufenden Ebene (50) verstellt.

4. Behandlungsinstrument nach Anspruch 3, dadurch gekennzeichnet, daß die genannte mechanische Vorrichtung aus zwei fest mit dem Gestell (3) verbundenen und je über ein Antriebsorgan (21, 22) angetriebenen Pleuelstange-Kurbelsystemen (18, 19) gebildet ist, die im Bereich der Enden jeder Pleuelstange (23, 24) mit einander zusammenwirken, um ein gemeinsames Gelenk (25) zu bilden, mit dem das Ende (9) der optischen Faser (5) fest verbunden wird, um sie in festgelegter Abszisse und Ordinate in einer Ebene zu verstellen.

5. Behandlungsinstrument nach Anspruch 4, dadurch gekennzeichnet, daß die Antriebsorgane (21, 22) jedes Pleuelstange-Kurbelsystems (18, 19) unabhängig gesteuert werden und deren Drehung eingestellt wird, um die sequenzielle Abtastung eines dem Bereich unter Untersuchung (2) ähnlichen Bereichs (50) zustandezubringen.

6. Behandlungsinstrument nach Anspruch 4, dadurch gekennzeichnet, daß die Antriebsorgane (21, 22) der Pleuelstange-Kurbelsysteme (18, 19) als "Schrittmotoren" ausgestaltet sind, die mit vorbestimmten analogen Stromgrößen gespeist werden, die es erlauben, die "Standardschritte" in mehrere "Mikroschritte" zu teilen, um die Positionierungsgenauigkeit zu erhöhen.

7. Behandlungsinstrument nach Anspruch 4, dadurch gekennzeichnet, daß jedes Pleuelstange-Kurbelsystem (18, 19) von einem Positionsgeber (30, 31) abhängig ist, der eine periodische Positionierungsinitialisierung der Antriebsorgane (21, 22) erlaubt.

8. Behandlungsinstrument nach Anspruch 1, das eine von einer vom Gestell (3) getragenen Fassung (14) unbeweglich gemachte und der optischen Hauptachse (11) entlang unter dem Ende (9) der optischen Faser (9) angeordnete Bündelungsoptik (10) aufweist, dadurch gekennzeichnet, daß die Mittel (15) zum Positionieren der Faser (5) und deren Bündelungsoptik (10) bezüglich des Bereich des Körpers unter Untersuchung (2) aus einem am Gestell (3) befestigten und auf die optische Hauptachse (11) zentrierten Stegenaggregat (33) gebildet sind, das an den Bereich des Körpers (2) anlehnt und Einstellmittel (34) zum Anpassen des Schießabstandes und, demzufolge, der Größe und der Einförmigkeit der Elementarabtastflecke (6) aufweist.

9. Behandlungsinstrument nach Anspruch 2, das eine von einer vom Gestell (3) getragenen Fassung (14) unbeweglich gemachte und der optischen Hauptachse (11) entlang unter dem Ende (9) der optischen Faser (9) angeordnete Bündelungsoptik (10) aufweist, dadurch gekennzeichnet, daß die Mittel zum Messen der Laserstrahlung aus einer von der Tragfassung (14) der Bündelungsoptik (10) gebildeten integrierenden Pseudokugel bestehen, die die indirekte Meßung der ausgestrahlten Gesamtkraft erlaubt, die Laserstrahlung, wovon ein Teil entnommen und von einem dem Laser angepaßten photoelektrischen Geber (37) gemessen wird, zurückstrahlt und verbreitet.

10. Behandlungsinstrument nach Anspruch 1, dadurch gekennzeichnet, daß es eine automatische Steuereinheit (49) für das genannte Instrument (1) umfaßt, die um eine elektronische Zentraleinheit, eine Zeitbasis und Nebenkreise, die außerdem die Überwachung der genannten mechanischen Abtastvorrichtung (18-27), die Meßung der Bestrahlungskraft, die Steuerung der synchronisierten Verschlußmittel, den Dialog mit dem Bedienungsmann erlauben, eingerichtet ist.

11. Behandlungsinstrument nach Anspruch 10, dadurch gekennzeichnet, daß der Abtastüberwachungskreis mindestens einen Speicher umfaßt, in den Daten eingegeben werden, um eine Laserstrahlschüßensequenzzu erlauben, um durch deren Aufschläge (17), durch in der Zeit genau wiederholbare Aneinanderreihung nebeneinanderliegender Elementarabtastflecke (6), eine geometrische Figur (29) zu decken.

12. Behandlungsinstrument nach Anspruch 11, dadurch gekennzeichnet, daß die geometrische Figur (29) über den Dialogkreis und den Abtastüberwachungskreis modulierbar ist.

13. Behandlungsinstrument nach Anspruch 11, dadurch gekennzeichnet, daß die geometrische Figur (29) als ein durch Aneinanderreihung von Elementarabtastflecken (6) eines Durchmessers zwischen 0,5 und 2 mm gebildetes regelmäßiges Sechseck ausgestaltet ist, dessen Diagonale zwischen 3 und 20 mm beträgt.

14. Behandlungsinstrument nach Anspruch 10, dadurch gekennzeichnet, daß die Steuereinheit (49) Mittel zum Aufheben der sequenziellen Abtastung von Laserstrahlschüßen, die getrennte Abtastflecke (6) bilden, umfaßt, um entweder eine automatische Abtastung nacheinanderfolgender nebeneinanderliegender Abtastflecke oder eine handbetätigte Abtastung zu erlauben.

FIG.1

FIG.2

60°

EP 0 265 470 B1

15

EP 0 265 470 B1

FIG.3

## FIG.4

## FIG.5

## FIG.6

## FIG.7

## FIG.8